Europäisches Patentamt

European Patent Office

Office européen des brevets

(19)

(11) Publication number: **0 401 813**
**A2**

# EUROPEAN PATENT APPLICATION

(21) Application number: 90110759.9

(22) Date of filing: 07.06.90

(51) Int. Cl.⁵: **C12Q 1/42, G01N 33/04**

(30) Priority: 08.06.89 US 363541

(43) Date of publication of application:
**12.12.90 Bulletin 90/50**

(84) Designated Contracting States:
**AT BE CH DE DK ES FR GB GR IT LI LU NL SE**

(71) Applicant: ADVANCED INSTRUMENTS, INC.
**1000 Highland Avenue**
**Needham Heights, Massachussetts**
**02194(US)**

(72) Inventor: **Rocco, Richard M.**
**6 Tilden Road**
**Canton, Massachusetts 02021(US)**
Inventor: **Bargoot, Frederick G.**
**24 Mellon Road**
**Wellesley, Massachusetts 02181(US)**

(74) Representative: **Eisenführ, Speiser & Strasse**
**Zweibrückenstrasse 17**
**D-8000 München 2(DE)**

(54) Method and device for the assay of alkaline phosphatase in dairy products.

(57) A method and device for the assay of alkaline phosphatase in dairy products. A sample of the dairy product is added to a self-indicating fluorometric substrate comprising a single orthophosphoric ester on a phenolic ring. After hydrolysis of the phosphate radical by the alkaline phosphatase in the sample, the substrate and the sample added thereto are exposed to electromagnetic radiation at a first wavelength. The increase in fluorescence at a second wavelength produced by the hydrolysis of the phosphate radical by the alkaline phosphatase is determined. This increase is linearly related to the activity of alkaline phosphatase present in the sample of the dairy product.

## Background of the Invention

This invention relates to a rapid, sensitive and specific method and device for the assay of alkaline phosphatase in dairy milk products, e.g. whole milk, skim milk, creams, and cheeses. Alkaline phosphatase (orthophosphoric monoester phosphohydrolase, alkaline optimum, ED 3.1.3.1) is an endogenous enzyme found in all mammalian milk. During pasteurization this enzyme is inactivated at temperatures slightly above those re-·quired for destruction of nonsporeforming microoganisms which are pathogenic to man. The measurement of this enzyme, therefore, has been used for over fifty years as an indicator of the completeness of pasteurization or of the contamination of the dairy product with raw un-pasteurized milk ("The Phosphatase Test for Pasteurized Milk", H.D. Kay and W.R. Graham, J. Dairy Res. 6:191-203, 1935). Raw un-pasteurized bovine milk contains over 2,000 International Units/Liter of alkaline phosphatase activity and after pasteurization, the level is reduced to less than 0.7 Units. The United States Federal Milk Ordinance for Grade A milk requires that all milk products intended for human consumption be tested for residual alkaline phosphatase and any product containing 0.7 Units or more cannot be offered for sale ("Grade A pasteurized Milk Ordinance", U.S. Department of Health and Human Services, Public Health Service, Food and Drug Administration, Publication No. 229, 1985 Revision).

Current methods for the measurement of alkaline phosphatase in dairy milk products are all based on colorometric procedures. An aliquot of the dairy product is incubated for 15 to 60 minutes with the substrate and the product formed by the action of alkaline phosphatase is removed from the milk matrix and read against standards in a spectrophotometer. The interfering turbidity and the low levels of enzyme found in the finished dairy product complicate the assay. Current techniques require organic solvent extraction of the formed enzymatic product, precipitation of interfering proteins and lipids with barium and zinc salts or dialysis for three hours into de-ionized water in order to overcome these problems. Modifications of these multistep and time-consuming colorometric procedures are the only methods approved for use in dairy milk products by the American Public Health Association (1985) and the International Dairy Federation (1971). ("Standard Methods for the Examination of Dairy Products", American Public Health Association, Wash. D.C., 1985; International Dairy Federation, B-1040 Brussels, Belgium, Publication FIL-IDF 63, issued 1971).

It is a general object of this invention to provide a simple procedure for the rapid (less than 3 minutes) and precise quantitative analysis of alkaline phosphatase activity in dairy milk products at exceedingly low levels (One Unit and below) which is simple and applicable to a wide variety of such products.

## Brief Description of the Invention

The present invention provides a method and device for the quantitative analysis of residual alkaline phosphatase activity in dairy milk products in a one-step assay. The specific embodiments of the invention involve the use of self-indicating fluorometric substrates in a direct assay requiring no separation of the fluorophore from the background milk turbidity.

Currently approved methods for alkaline phosphatase in dairy milk products are based on measurement of the liberated product due to the action of the enzyme on a defined substrate in a specified time period. For example, mcg phenol released from phenyl phosphate substrate/15 minutes/ml or gram of sample (The American Public Health Association and International Dairy Federation Procedures). The universal cutoff value for unacceptable milk products is 1.0 mcg phenol/15 min/ml. In International Units of activity this is equivalent to 0.7 IU/L or gram of sample. Products containing 0.7 or more of alkaline phosphatase activity cannot be sold for human consumption. The value of 0.7 IU/L has been chosen because it represents the activity obtained with current methods on fluid milk products contaminated with 0.1% (v/v) raw or unpasteurized milk. At the decision level of 0.7 IU/L, current methods have a coefficient of variation under controlled conditions of up to 13.4% with a lower detection limit approaching 0.3 IU/L ("Collaborative Study of Rapid Methods For Alkaline Phosphatase in Milk and Cream", G.K. Murthy and J.T. Peelen, J. Food Protection 42:800-803, 1979).

Using the fluorometric assay of the present invention on dairy milk products containing 0.7 IU/L alkaline phosphatase activity (for example, containing 0.1% (v/v) raw unpasteurized milk) the coefficient of variation from repeated analysis was less than 5% and a lower detection limit approaching 0.01 IU/L was achieved.

## Detailed Description of the Invention

The method and device of the invention utilizes various self-indicating fluorometric substrates for analysis of alkaline phosphatase in dairy milk products. These substrates contain in their structure a single orthophosphoric ester on a phenolic ring. These compounds are non-flourescent when excited at wavelengths above 230nm, that is, give off minimal fluorescence in the 250 to 700 nm range. Upon hydrolysis of the phosphate radical by alkaline phosphatase, the compound exhibits a quantitative fluorescence which is linearly related to the activity of alkaline phosphatase present in the sample. Fluorometric substrates which can be used include:
4-methylumbelliferyl phosphate; 7(3-phenylcoumarinoyl) phosphate;
3-(2-(5-chlorobenzoxazolyl)4-cyano-7-courmarinoyl-phosphate;
6-bromo-3-hydroxy-2-naphthyl-O-anisidine phosphate;
2'4'-dimethyl-3-hydroxy-2-naphthanilide phosphate;
3-O-methyl-fluorescein phosphate.
Among the buffers which can be used are 2-amino-2-methyl-1-propanal; diethanolamine; ethylaminoethanol; and tris(hydroxymethyl)-aminomethane.

Among the advantages of the invention are:

1. Alkaline phosphatase levels of activity in milk products can be measured with greater sensitivity than prior methods by a factor of 10-100 times.

2. The fluorometric assay of alkaline phosphatase activity is more specific and reproducible than prior methods. No organic solvent extraction, barium-zinc precipitation or dialysis steps are required.

3. The fluorometric assay is more rapid than prior methods. Total assay time for a quantitative assay is under three minutes compared to one and one-half to four hours for current methods.

4. Automation of the fluorometric procedure is possible, thus allowing hundreds of finished dairy milk products to be tested in shorter periods of time prior to being shipped to retail outlets. The benefits are that finished dairy milk products will spend less time in storage at the manufacturing facility.

5. The improved sensitivity of the fluorometric assay can assist the dairy industry in investigating with greater precision current problems in assessing milk quality. For example, the detection of reactivated alkaline phosphatase in ultra high temperature pasteurized products and detection of low levels of alkaline phosphatase produced by the growth of bacteria during the storage of finished dairy milk products, especially cheeses, is easily accomplished.

6. The improved sensitivity of the fluorometric assay provides for the detection of low levels of alkaline phosphatase activity in food and dairy products which has not been possible before. For example, the detection of fecal contamination of grains, cereals, and spices.

The present invention will be further clarified with reference to the following illustrative embodiments, which are intended to be exemplary only, and not to limit the scope of the invention:

Example

Sample preparation: The use of fluorometric substrates as embodied in this invention obviates the need for special sample preparation or concentration steps prior to analysis or removal of the product formed due to the enzyme action as is required in all prior assays. Whole regular milk, skim milks, low-fat milks, flavored milks, and creams can be sampled directly after being well mixed. Buttermilks, sour creams, and creamed cottage cheeses are sampled after brief homogenization in a mechanical blender in order to ensure uniformity of the sample. Concentrated and dry-milk products need only be reconstituted with appropriate amounts of water. Butter may be melted at 40°C; mixed well and an aliquot directly removed from analysis. Solid foods such as cheese, grains, cereals, and spices are extracted with 4 volumes of neutralized normal butyl alcohol 7.5% (v/v) in laboratory grade water. The extract is centrifuged for 10 minutes at 2500 x g and an aliquot removed from the middle layer for direct analysis.

Example 1

Procedure: Alkaline phosphatase substrate (example, 4-methylumbelliferyl phosphate) is dissolved in 1mM at diethanolamine buffer 1.0M pH 10.0 with 0.23mM magnesium chloride and 0.005% sodium azide. This working substrate is aliquoted into 1cm square cuvettes and pre-warmed in a water bath to 38°C for 2 to 3 minutes. Performance of the assay involves the addition of 0.1 ml sample to the substrate, mixing by inversion several times and placing the cuvette into a suitable thermo-cuvetted fluorometer. Excitation and emission light should be set at appropriate wave lengths for the substrate being used (example, 365 nm excitation and 447 nm emission). The increase in fluorescence caused by the hydrolysis of the phosphate radical by alkaline phosphatase should be monitored for two to three minutes. A strip chart recorder was used for this purpose. The increase in fluorescence/minute/0.1ml of sample is then converted to micromoles product formed/minute/0.1ml sample by comparison to a calibration curve prepared by serial dilution of the liberated fluorophore (example, 4-methyl-umbelliferone). Multiplication of micromoles product formed/minute/0.1ml sample is converted by simple multiplication to mU/L alkaline phosphatase activity, where activity is defined as the number of micromoles of substrate converted/minute/liter of sample at specified temperature.

Example 2

Procedure: Alkaline phosphatase substrate (example; 4-methylumbelliferyl phosphate) is dissolved at 1mM in diethanolamine buffer 1.0M pH 10.0 with 0.23mM magnesium chloride and 0.005% sodium azide. This working substrate is then aliquoted into a porous polyethylene membrane with average pore size of 65 to 75 microns. Performance of the assay involves addition of a measured amount of fluid milk product to the membrane. The membrane with substrate and milk sample is then placed into a thermostated (38°C) cuvette chamber of a standard fluorometer fitted with a front face surface adapter. After a few minutes temperature equilibration period the increase in fluorescence caused by the hydrolyses of the substrate in the membrane by the added alkaline phosphatase is monitored for two to three minutes.

The use of this test system in a membrane format confers three advantages over a conventional liquid system. First, front face surface fluorometry is more linear in response than conventional liquid in a cuvette system. This extends the dynamic range of the assay and reduces the need for dilution of elevated samples. Second, the membrane system described reduces the quenching effects found in turbid liquid systems. In the front surface system the excitation light is not partially lost within the matrix of the liquid system because it is interacting at the surface of the membrane. In turn, collection of emitting light is facilitated by collection from the surface rather than have to be collected through a turbid solution which causes some loss of energy. Third, the use of a membrane system reduces the amount of reagent needed for each assay and therefor the amount of sample required. The size of the membrane used is limited only by the configuration of the fluorometer and the membrane adapter used in the chamber holder. In this example, membranes measuring 13mm wide by 45mm long by 3mm thick were used.

Having described in detail a preferred embodiment, it will now be apparent to those skilled in the art that numerous modifications can be made therein without departing from the scope of the invention as defined in the following claims:

Claims

1. A method for the assay of alkaline phosphatase in a sample of a dairy product, said method comprising the steps of:
   A. adding the sample of dairy product to a self-indicating flurormetric substrate, said substrate comprising a single orthophosphoric ester on a phenolic ring;
   B. allowing hydrolysis of the phosphate radical by alkaline phosphatase in said sample;
   C. exciting the self-indicating fluorometric substrate and the sample of the dairy product added thereto by exposure to electromagnetic radiation at a first wavelength; and,
   D. determining the increase in fluorescence at a second wavelength produced by the hydrolysis of the phosphate radical by the alkaline phosphatase in the sample, said increase being linearly related to the activity of alkaline phosphatase present in the sample of the dairy product.

2. The method of claim 1 wherein the fluorometric substrate comprises 4-methylumbelliferyl phosphate.

3. The method of claim 1 wherein the fluorometric substrate comprises 7(3-phenylcoumarinoyl) phosphate.

4. The method of claim 1 wherein the fluorometric substrate comprises 3-(2-(5-chlorobenzoxazolyl) 4-cyano-7-courmarinoyl-phosphate.

5. The method of claim 1 wherein the fluorometric substrate comprises 6-bromo-3-hydroxy-2-naphthyl-O-anisidine phosphate.

6. The method of claim 1 wherein the fluorometric substrate comprises 2'4'-dimethyl-3-hydroxy-2-naphthanilide phosphate.

7. The method of claim 1 wherein the fluorometric substrate comprises 3-O-methyl-fluorescein phosphate.

8. The method of claim 1 wherein the self-indicating substrate fluoresces at wavelengths below 230 nm with minimal fluorescence in the 250 to 700 nm range prior to hydrolysis of the phosphate radical by alkaline phosphatase.

9. The method of claim 1 wherein said fluorometric substrate is held within the pores of a porous membrane.

10. A device for indicating alkaline phosphatase activity in dairy products comprising:
   (1) a porous membrane; and,
   (2) a self-indicating fluorometric substrate comprising a single orthophosphoric ester on a phenolic ring, said substrate being held within the pores of said membrane.

11. The device of claim 10 wherein the fluorometric substrate comprises 4-methylumbelliferyl phosphate.

12. The device of claim 10 wherein the fluorometric substrate comprises 7(3-phenylcoumarinoyl) phosphate.

13. The device of claim 10 wherein the fluorometric substrate comprises 3-(2-(5-chlorobenzoxazolyl) 4-cyano-7-courmarinoyl-phosphate.

14. The device of claim 10 wherein the fluorometric substrate comprises 6-bromo-3-hydroxy-2-naphthyl-O-anisidine phosphate.

15. The device of claim 10 wherein the fluorometric substrate comprises 2′4′-dimethyl-3-hydroxy-2-naphthanilide phosphate.

16. The device of claim 10 wherein the fluorometric substrate comprises 3-O-methyl-fluorescein phosphate.

17. The device of claim 10 wherein said porous membrane is a polyethylene membrane.

18. The device of claim 10 wherein the polyethylene membrane has an average pore size of 65 to 75 microns.